# EUROPEAN PATENT SPECIFICATION

(11) **EP 0 571 058 B1**
(45) Date of publication and mention of the grant of the patent: **11.09.1996**
(21) Application number: 93250138.0
(22) Date of filing: 12.05.1993
(51) Int. Cl.: A61B 17/00

(54) **Coupling for surgical instruments**
Kupplungseinrichtung für chirurgische Instrumente
Dispositif d'accouplement pour instruments chirurgicaux

(30) Priority: 21.05.1992 DE 4216874
(43) Date of publication of application: 24.11.1993
(73) Proprietor: ETHICON INC., Somerville, New Jersey 08876-0151 (US)
(72) Inventor: Tkaczyk, Peter, W-7900 Ulm (DE)
(74) Representative: UEXKÜLL & STOLBERG

(56) References cited:
- EP-A- 0 495 633
- DE-A- 3 343 867
- DE-U- 8 709 151
- US-A- 2 113 246
- US-A- 4 043 323

## Description

The invention relates to a coupling for the releasable connection of a surgical instrument working with movable parts for laparoscopic operations which comprises an instrument tube with a push rod displaceably guided therein, with a grip which comprises a housing and an adjustment element which is displaceably guided therein and driven through actuation of the grip.

In modern minimally invasive surgery, no opening of the operation zone is carried out, but the instruments needed for the procedure are introduced into the abdominal cavity through one or more cannulae and guided under the control of an endoscope likewise introduced into the abdominal cavity or under X-ray observation for the performance of the operation. Instruments of various types, for example forceps, needle holders, scissors, clip-setting apparatus etc., are needed to perform the operations. Such instruments are held by the operator at a grip and actuated through a pressure or closure movement at the grip. The actuation performed at the grip is transmitted through the extended laparoscopic instruments to the movable instrument parts lying at the distal end, such as for example the jaws of a pair of forceps, via a push- or pull rod or a rope device.

It is desirable that the actual instrument and the actuation grip be provided as separate parts, as the instruments are frequently provided for one-off use or for use on one patient while the actuation grip is sterilizable and can be used repeatedly. It is also advantageous to keep a large range of instruments ready and provide whichever one is desired with an actuation grip, whereby the overall cost is reduced when a grip does not have to be procured and kept ready for every instrument. The use each time of a new instrument for every patient also has advantages from the point of view of hygiene, removing the risk of infection arising from inadequately cleaned and sterilized instrument parts which come into direct contact with the operation area.

Used as actuation grip for the handling of laparoscopic instruments are, for example, scissor- or forceps-like grip levers whose closure movement is transmitted onto an adjustment element into a movement in the direction of the instrument's longitudinal axis and further onto a push- or pull rod in the instrument. Other typical grip designs use grip disks which are displaceable against each other, one of which lies in the surgeon's palm and the other is gripped with the fingers and pushed against the first, this pushing movement being convertible, through a transmission provided in the housing of the grip, into a pulling movement onto an adjustment element. To connect such actuation grips to a laparoscopic instrument, the housing of the actuation grip and the instrument tube of the laparoscopic instrument on the one hand, and the driven adjustment element in the actuation grip and the push rod on the other, must be connected, the push rod being guided in the instrument tube and transmitting the force exerted on the grip and transmitted via the adjustment element onto the movable instrument members.

A releasable connection between a grip and a laparoscopic instrument is described for example in DE-GM 87 09 151. In the known device, a grip piece is connected to an instrument or catheter tube by engaging notches secured at the grip piece in a thicker section at the proximal end of the instrument tube, thus holding the thicker section fast at the grip piece. The notches are bendable and can be bent back so far that they release the thicker section, whereupon the instrument can be removed in axial direction from the grip piece. The bending back of the notches takes place through displacement of a sleeve which is guided over the notches and the grip piece and which engages at guide surfaces at the notches and bends back the notches when the sleeve is displaced to release the instrument tube. The described coupling mechanism contains no provision for a simultaneous connection of the driven adjustment element in the grip with the push rod or the draw rope in the instrument tube.

DE-A-33 43 867 discloses a releasable coupling for a surgical instrument wherein the proximal end region of an instrument tube can be placed into a holding meter which is fixed to a gripping part. A slotted sleeve, which is rotateably mounted on the holding member, is used to secure the instrument tube at the holding member. Inside the instrument tube, a push rod is displaceably guided, the proximal end of which protrudes from the instrument tube. The proximal end of the push rod has to be connected to another gripping part which can be moved against the first gripping part in order to actuate the instrument.

The object of the invention is to provide a releasable coupling between a laparoscopic instrument working with movable instrument members and an actuation grip, the coupling being both simple to execute in design terms and simply and safely manageable for the surgeon upon joining together.

This object is achieved by the coupling with the features of claim 1. Advantageous versions of the invention are listed in the dependent claims.

The coupling according to the invention provides, for the connection of the driven adjustment element in the grip and the push rod in the instrument tube, a first locking element which is arranged at the proximal end of the push rod and is laterally insertable into an appropriately shaped first recess in the adjustment element in order to secure the push rod against axial movement in the adjustment element. To connect the grip housing to the instrument tube, there is present at the distal end of the housing a second recess, into which a second locking element projecting at the proximal end at the periphery of the instrument tube is laterally insertable, in order to secure the instrument tube against axial movement against the housing. The housing and the recesses are so designed that they are so wide open in at least one side zone, i.e. in the direction transverse to the instrument or longitudinal grip axis, that the instrument tube and the push rod are laterally introducible through the open side zone into the housing and the locking elements are simultaneously insertable into their respective recesses. It is thereby possible, in a single movement, to insert the laparoscopic instrument from a lateral direction into the grip housing and simultaneously create, between the instrument tube and the grip housing on the one hand and the push rod and the adjustment element of the grip on the other, a coupling secured against axial movement. In order to secure the instrument tube and the push rod in their respective recesses against lateral movement and simultaneously close the laterally open zones through which the instrument tube with the push rod was inserted into the housing and the appropriate recesses, there is provided a displaceable closing sleeve which, after the instrument has been pushed into the grip, is moved over the open lateral zones in order to enclose the grip housing in this zone and thereby prevent lateral mobility of the locking elements.

In an advantageous version, the first locking element is formed by a first plate secured transverse to the rod at the proximal end. The first plate has a rounded outer edge which is so shaped that, following insertion into the first recess, which is shaped to match the first plate, it supplements the open lateral zone of the housing. As a result, the closing sleeve which has been pushed over lies outside against the housing and, in the latter's open zone, against the outer edge of the first plate, in order thus to secure the latter against lateral movement.

Starting from the outer edge, the connecting side edges of the first plate run onto one another, so that a tapering profile results. Thanks to this configuration the first locking element can be easily inserted into the suitably shaped first recess and aligns itself in the process.

In a further preferred version, the second locking element is formed by a second plate projecting laterally at the proximal end of the instrument tube. This second plate likewise comprises, advantageously transversely to the instrument tube, a tapering profile and a rounded outer edge. In this way the second locking element can also be inserted easily and in a self-aligning manner into the suitably shaped second recess.

The configuration of grip and instrument according to the invention leads to a quickly releasable and closable coupling, with the surgeon or assistants holding the instrument in one hand and the grip in the other and then pushing the two laterally into each other with a single movement and then securing the coupling by pushing the closing sleeve over it.

It is an advantage of the invention that the insertion of the instrument takes place under the full visual supervision of the user and secure handling is thereby ensured.

The invention is described in more detail below, with reference to an embodiment, in the drawings; these show:
- Figure 1:: a section through a grip and the proximal end of a coupled-on instrument;
- Figure 2:: a section along the line 2 from Figure 1; and
- Figure 3:: a section along the line 3 from Figure 1.

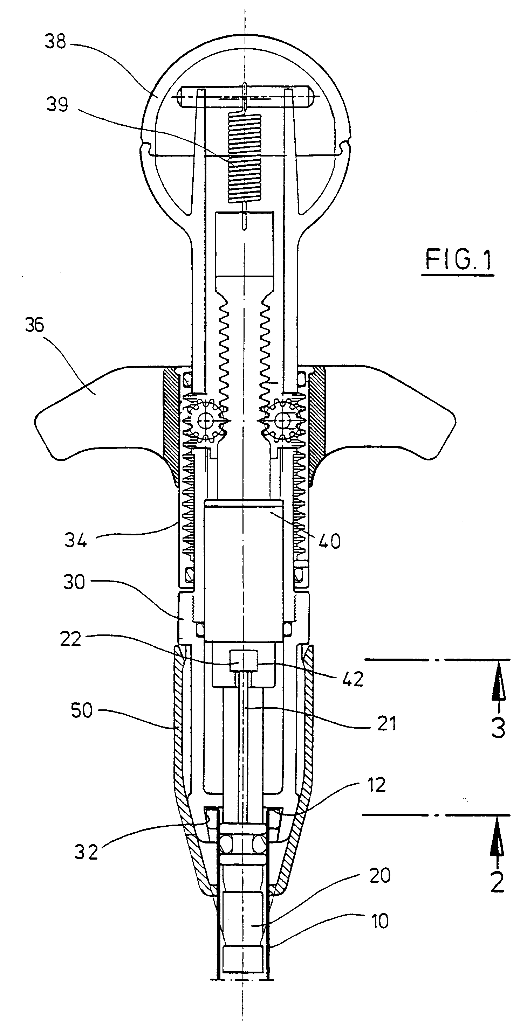

Figure 1 shows a grip and the proximal end of a coupled-on instrument in section. The grip has a rearward, spherical element 38 which lies in the surgeon's palm, and also, further forward, projecting grip elements 36 which are grasped with the fingers and, for actuation of the grip, pushed by the surgeon against the spherical element 38. The longitudinal movement effected by this is converted via toothed rods, toothed wheels and further toothed rods into a pulling movement of the adjustment element 40 with respect to the housing 30 of the grip. Upon actuation of the grip, the adjustment element 40 is pushed forward, i.e. moved to the left in the representation of Figure 1. The adjustment element 40 is provided with a first recess 42 at its distal end. A first locking element 22, which is secured at the distal end of a push rod 20, 21, is inserted into the first recess 42 and, in respect of axial movement, i.e. movement in the longitudinal direction of the push rod, is in form-locking engagement with the first recess 42 at the adjustment element 40.

The push rod 20 is displaceably guided in an instrument tube 10 and comprises a cross profile at 21. The instrument tube 10 is provided at its proximal end with a wider section which serves as second locking element 12. The second locking element 12 fits into a suitably shaped second recess 32 at the distal end of the housing 30.

To fix the locking elements in their respective recesses, a closing sleeve 50 is pushed over the front zone of the housing 30 and thus covers the first locking element 22 in the first recess 42 and the second locking element 12 in the second recess 32 and thereby secures the locking elements against movement in lateral direction, i.e. in the direction out from the respective recesses.

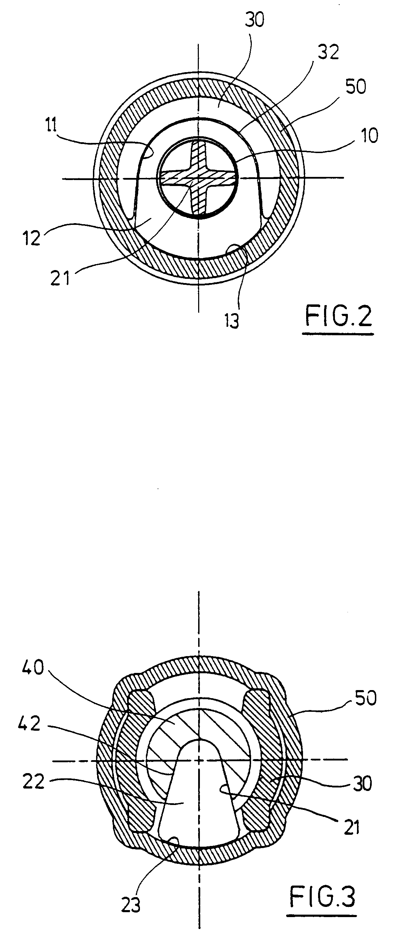

Figure 2 shows a sectional representation along the line 2 in Figure 1 in the zone of the second recess 32 and of the second locking element 12. The wider section at the proximal end of the instrument tube 10 lies as a second locking element 12 in the second recess 32 at the front end of the housing 30. The second locking element 12 comprises a rounded outer edge 13 and tapering side edges 11 starting from same. The second locking element 12 lies with the side edges 11 against the complementarily shaped second recess 32. The housing 30 has a laterally open configuration, so that the instrument tube 10 with the second locking element 12 is laterally insertable into the housing 30 into the second recess 32 when closing sleeve 50 is opened.

The push rod 20 has the shape of a cross profile at 21 in the zone guided in the housing. The cross profile of the push rod 20 continues to almost as far as its proximal end, where the first locking member 22 is then connected to a thinner zone.

In Figure 3 the coupling is shown in the zone of the first locking element 22 in lateral section along the line 3 from Figure 1. The first locking element 22 comprises a rounded outer edge 23 and side edges 21 which run onto one another and lie in the first recess 42 of the adjustment element 40. Pushed over the housing 30 is the closing sleeve 50 which, on the outside, lies against the outer edge 23 of the first locking element 22. The closing sleeve 50 is preferably made from solid material such as e.g. plastics and matched to the shape of the housing 30 so as to lie in locking manner against the housing 30 when the sleeve is closed.

Upon actuation of the grip, the first locking element 22 slides with its outer edge 23 along the inside of the closing sleeve 50.

The coupling is released by pushing the closing sleeve 50 off from the housing 30 along the instrument tube 10 and then withdrawing the instrument tube 10 and the push rod 20 guided therein from their recesses through lateral movement, i.e. movement downwards in the representation of Figures 2 and 3. In the zone between the first recess 42 and the second recess 32, the housing is laterally opened at least so wide that the push rod 20 fits through the opened zone. In the zone before the second recess 32, the housing must be opened at least so wide that the instrument tube 10 is introducible through the opened zone.

## Claims

1. Coupling for releasably connecting a surgical instrument working with movable parts for laparoscopic operations which comprises an instrument tube (10) with a push rod (20) displaceably guided therein, with a grip which comprises a housing (30) and an adjustment element (40) which is displaceably guided therein and driven through actuation of the grip, wherein
a first locking element (22) is provided at the proximal end of the push rod (20) and a second locking element (12) at the proximal end of the instrument tube (10),
a first recess (42) is provided at the distal end of the adjustment element (40) and a second recess (32) at the distal end of the housing (30), the first and the second recess and the housing (30) being laterally opened so wide in at least one side zone that the first (22) and the second locking element (12) are laterally insertable in transverse direction relative to the longitudinal direction of the push rod (20) into the first (42) and the second recess (32), respectively, and are secured there against axial movement, and
a closing sleeve (50), displaceable in longitudinal direction, can be moved over the instrument tube (10) and the housing (30) in the zone of the first (42) and the second recess (32) in order to secure the first (22) and the second locking element (12) against lateral movement in the first (42) and the second recess (32).

2. Coupling according to claim 1, **characterized in that** the first locking element (22) is formed by a first plate which runs transversely to the push rod (20) and is secured at the proximal end, the profile of said plate comprising tapering side edges (21) starting from a rounded outer edge (23) which, after insertion of the first plate into the first recess (42), which is suitably shaped to match the first plate for form-locking engagement, essentially continues the contour of the housing (30).

3. Coupling according to one of the previous claims, **characterized in that** the second locking element (12) is formed by a second plate projecting at the proximal end of the instrument tube (10), which plate comprises, transversely to the instrument tube (10), a tapering profile and a rounded outer edge (13) which, after insertion of the second plate into the second recess (32), which is suitably shaped to match the second plate for form-locking engagement, supplements the contour of the housing (30) in the laterally opened zone.

## Patentansprüche

1. Kupplung zur lösbaren Verbindung eines mit beweglichen Teilen arbeitenden chirurgischen Instruments für laparoskopische Operationen, das ein Instrumentenrohr (10) mit einer darin verschieblich geführten Schubstange (20) aufweist, mit einem Griff welcher ein Gehäuse (30) und ein darin verschieblich geführtes, durch Betätigung des Griffs angetriebenes Stellelement (40) aufweist, wobei
am proximalen Ende der Schubstange (20) ein erstes Riegelstück (22) und am proximalen Ende des Instrumentenrohres (10) ein zweites Riegelstück (12) vorgesehen ist,
am distalen Ende des Stellelementes (40) eine erste Ausnehmung (42) und am distalen Ende des Gehäuses (30) eine zweite Ausnehmung (32) vorgesehen ist, wobei die erste und die zweite Ausnehmung und das Gehäuse (30) in wenigstens einem Seitenbereich seitlich soweit geöffnet gestaltet sind, daß das erste (22) und das zweite Riegelstück (12) seitlich, in Richtung quer zur Längrichtung der Schubstange (20) in die erste (42) bzw. die zweite Ausnehmung (32) einschiebbar und dort gegen axiale Bewegung gesichert sind, und
eine in Längsrichtung verschiebbare Verschlußhülse (50) über das Instrumentenrohr (10) und das Gehäuse (30) im Bereich der ersten (42) und der zweiten Ausnehmung (32) schiebbar ist, um das erste (22) und das zweite Riegelstuck (12) gegen seitliche Bewegung in der ersten (42) und der zweiten Ausnehmung (32) festzuhalten.

2. Kupplung nach Anspruch 1, dadurch gekennzeichnet, daß das erste Riegelstück (22) durch eine quer zur Schubstange (20) verlaufende und am proximalen Ende befestigte erste Platte gebildet ist, deren Profil sich verjüngende Seitenkanten (21) ausgehend von einer abgerundeten Außenkante (23) aufweist, welche nach Einschieben der ersten Platte in die an die erste Platte zum formschlüssigen Eingriff angepaßt geformte erste Ausnehmung (42) im wesentlichen die Kontur des Gehäuses (30) fortsetzt.

3. Kupplung nach einem der vorhergehenden Ansprüche, dadurch gekennzeichnet, daß das zweite Riegelstück (12) durch eine am proximalen Ende des Instrumentenrohres (10) vorspringende zweite Platte gebildet ist, die quer zum Instrumentenrohr (10) ein sich verjüngendes Profil und eine abgerundete Außenkante (13) aufweist, die nach Einschieben der zweiten Platte in die an die zweite Platte zum formschlüssigen Eingriff angepaßt geformte zweite Ausnehmung (32) die Kontur des Gehäuses (30) im seitlich geöffneten Bereich ergänzt.

## Revendications

1. Accouplement pour relier de manière libérable un instrument chirurgical agissant à l'aide de parties mobiles pour des opérations de laparoscopie, qui comporte un tube d'instrument (10), muni d'une tige de poussée (20) pouvant se déplacer de manière guidée dans celui-ci, à une poignée qui comporte un boîtier (30) et un élément d'ajustement (40) qui peut se déplacer de manière guidée dans celui-ci et entraîné par actionnement de la poignée, dans lequel :
un premier élément de verrouillage (22) est agencé s l'extrémité proximale de la tige de poussée (20) et un second élément de verrouillage (12) est agencé à l'extrémité proximale du tube d'instrument (10),
un premier évidement (42) est agencé à l'extrémité distale de l'élément d'ajustement (40) et un second évidement (32) est agencé à l'extrémité distale du boîtier (30), le premier et le second évidement ainsi que le boîtier (30) étant ouverts latéralement de manière si large, dans au moins une zone latérale, que le premier (22) et le second élément de verrouillage (12) peuvent être insérés latéralement dans une direction transversale par rapport à la direction longitudinale de la tige de poussée (20) dans le premier (42) et le second évidement (32), respectivement, et y sont immobilisés vis-à-vis d'un mouvement axial, et
un manchon de fermeture (50) pouvant se déplacer dans la direction longitudinale, peut être déplacé par-dessus le tube d'instrument (10) et le boîtier (30) dans la zone du premier (42) et du second évidement (32) afin d'immobiliser le premier (22) et le second élément (12) de verrouillage à l'encontre d'un mouvement latéral dans le premier (42) et le second évidement (32).

2. Accouplement selon la revendication 1, caractérisé en ce que le premier élément de verrouillage (22) est formé par une première place qui s'étend transversalement à la tige de poussée (20) et est immobilisée à l'extrémité proximale, le profil de ladite plaque comportant des bords latéraux (21) inclinés commençant à partir d'un bord extérieur arrondi (23) qui, après l'insertion de la première plaque dans le premier évidement (42), qui est formé de manière appropriée pour apparier la première plaque pour mise en prise de verrouillage, prolonge pratiquement le contour du boîtier (30).

3. Accouplement selon l'une quelconque des revendications précédentes, caractérisé en ce que le second élément de verrouillage (12) est formé par une seconde plaque faisant saillie à l'extrémité proximale du tube d'instrument (10), laquelle plaque comporte, transversalement au tube d'instrument (10), un profil évasé et un bord extérieur arrondi (13) qui, après l'insertion de la seconde plaque dans le second évidement (32), qui est formé de manière appropriée pour apparier la seconde plaque pour mise en prise de verrouillage, complète le profil du boîtier (30) dans la zone ouverte latéralement.
